# EUROPEAN PATENT SPECIFICATION

(11) **EP 1 035 776 B1**
(45) Date of publication and mention of the grant of the patent: **26.03.2003**
(21) Application number: 98966254.9
(22) Date of filing: 04.12.1998
(51) Int. Cl.: A01N 63/04, A01H 15/00, C12N 1/14

(54) **FUNGI OF THE GENUS PIRIFORMOSPORA**
PILZE DER GATTUNG PIRIFORMOSPORA
CHAMPIGNONS DU GENRE PIRIFORMOSPORA

(30) Priority: 05.12.1997 EP 97121440
(43) Date of publication of application: 20.09.2000
(73) Proprietor: Max-Planck-Gesellschaft zur Förderung der Wissenschaften e.V., Berlin (DE)
(72) Inventor: FRANKEN, Philipp, D-35085 Ebsdorfergrund (DE); VARMA, Ajit, MPI für terrestrische Mikrobiologie, D-35043 Marburg (DE)
(74) Representative: VOSSIUS & PARTNER
(86) International application number: EP9807913
(87) International publication number: WO99029177

(56) References cited:
- EP-A- 0 485 229
- US-A- 5 178 642
- VERMA, SAVITA ET AL: "Piriformospora indica, gen. et sp. nov., a new root-colonizing fungus." MYCOLOGIA, (SEPT.-OCT., 1998) VOL. 90, NO. 5, PP. 896-903. ISSN: 0027-5514., XP002099843
- DATABASE WPI Section Ch, Week 9344 Derwent Publications Ltd., London, GB; Class C07, AN 93-347597 XP002099845 & JP 05 252999 A (PENTEL KK & RIKAGAKU KENKYUSHO), 5 October 1993

## Description

The present invention relates to fungi of the genus Piriformospora, to chlamydospores and mycelia of such fungi, to cultures and culture media of said fungi, as well as to substrates and compositions comprising the fungi, chlamydospores, mycelia or culture media for promoting development, growth, health, nutrition of plants, regeneration of plants and for promoting germination of plant seeds.

Fungi interact with plants as pathogens or benefactors and therefore strongly influence the yields in agro-forestry and flori-horticulture. The most wide spread symbionts are the arbuscular mycorrhizal fungi (AMF) which occur on more than 80% of all land plants (Newman, New Phytol. 106 (1987), 745-751 and Tester, Can. J. Bot. 65 (1987), 419-431). As mutualists, they are able to improve the growth of crops on poor soils at the same time reducing the input of expensive and polluting chemical fertilizers and pesticides (Gianinazzi, Crit. Rev. Biotechnol. 15 (1995), 305-311 and Bethlenfalvay in "Mycorrhizae in sustainable agriculture"; eds. Bethlenfalvay and Linderman, American Society of Agronomy, Madison, Wisconsins, USA (1992), 1-27). Unfortunately, AMF cannot be grown in pure culture. Many studies are therefore very difficult to carry out which slows down any fundamental research and more seriously, their biotechnological application.

Therefore, the technical problem underlying the present invention is the provision of fungi which are capable to interact beneficially with plants and which are more easy to handle than the known arbuscular mycorrhizal fungi. This problem has been solved by the provision of the embodiments as defined in the claims.

Thus, the present invention relates to a fungus of the genus Piriformospora having at least the following features:
(a) the hyphae are septated;
(b) the septal pores consist of dolipores with continuous parenthosomes;
(c) the mycelium produces at the tips of the hyphae chlamydospores which emerge apically;
(d) the chlamydospores have a pear shaped structure; and
(e) the fungus has the ability to interact with living plant roots.

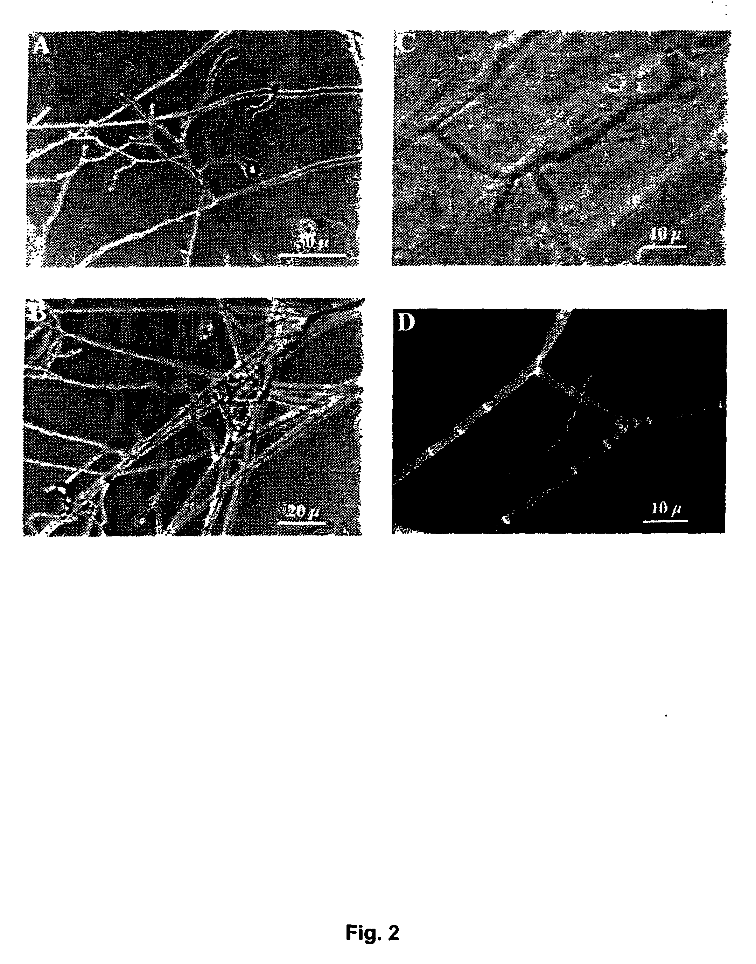

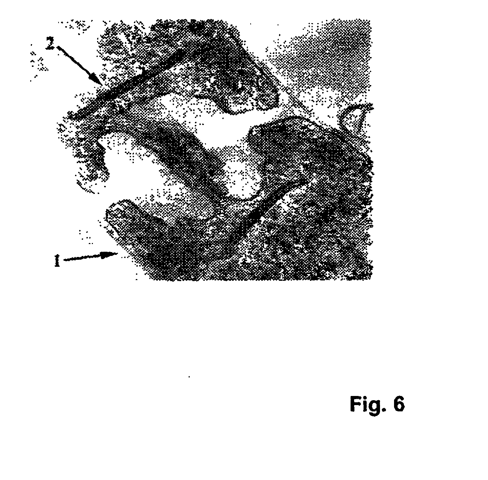

A fungus having the above-mentioned characteristics was isolated from a desert soil in north western India during routine monosporic inoculum preparation of an arbuscular mycorrhizal fungus. It was found that this fungus does not belong to any of the known geni of fungi and, thus, constitutes a novel genus which was called Piriformospora due to the shape of the chlamydospores produced by the fungus. Furthermore, it was found that the fungus can be cultivated easily and has serveral advantageous effects on plants when interacting with their roots. As indicated above, a characteristic of the fungus of the present invention is that the hyphae are septated. Preferably, this septation is irregular (Fig. 2D). In this case, it is possible that the single compartments of the hyphae contain more than one nucleus.
The cell walls of the fungus according to the invention are preferably very thin and more preferably show multilayered structures (Fig. 6, arrow 1). Typically, the hyphae of the mycelium range in diameter from about 0.7 to about 3.5 mm. The dolipores of the septal pores are preferably very dominant with a multilayered crosswall and more preferably also have a median swelling mainly consisting of electron transparent material. Most preferably the electron transparent layers of the cross walls extend deep into the median swellings but do not fan out. It is preferred that in the median section of the septal pores the parenthosomes are straight and, more preferably, have the same diameter as the corresponding dolipore. Furthermore, the parenthosomes preferably do not have any kind of pores, i.e. they are flat discs without any perforation (Fig. 6, arrow 2). The parenthosomes preferably consist of an electron dense outer layer and a less dense inner layer.

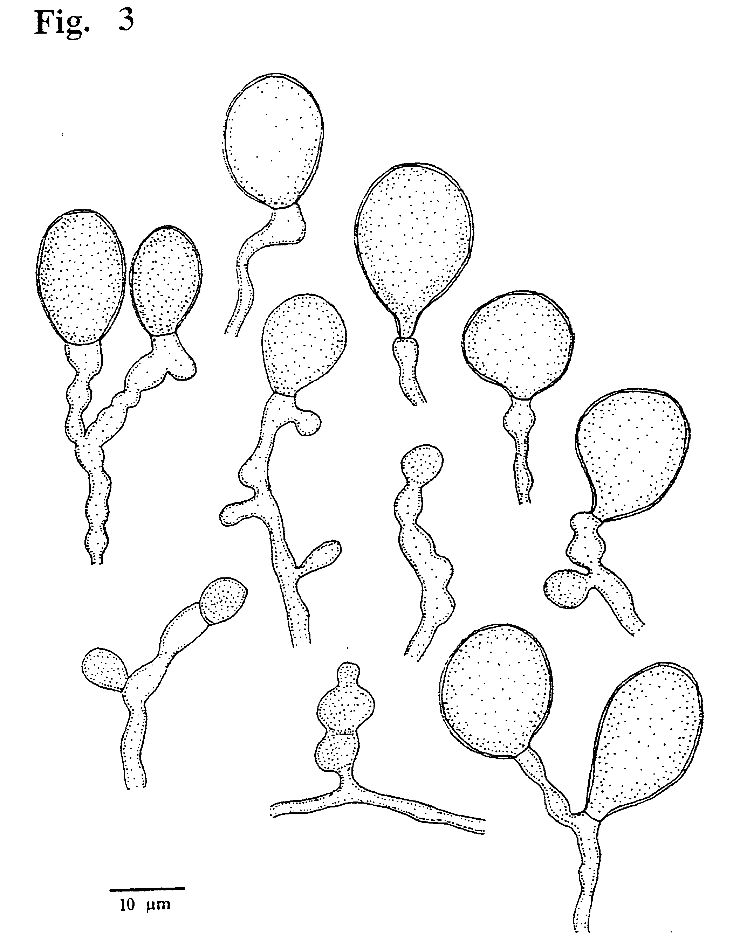

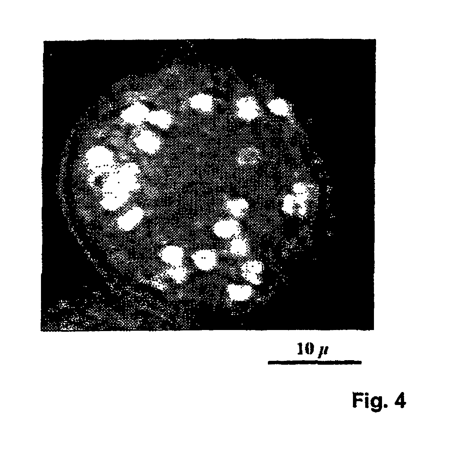

This inner layer preferably shows an inconspicious dark line in the median region. More preferably the parenthosomes are in contact with the endoplasmic reticulum. The membranes of the endoplasmic reticulum are typically found mostly near the dolipore.
The spores of the fungus, which are chlamydospores are preferably formed from thin-walled vesicles at the tips (apex) of the hyphae (Fig. 3), preferably at the apex of undifferentiated hyphae. They may appear singly or in clusters. The chlamydospores of the fungus of the present invention have a typical pear shaped structure. Preferably, very young spores have one-layered thin, hyaline walls. At maturity, they preferably have at least one of the following features: they are thick, double walled, smooth and/or pale yellow. More preferably, cytoplasm in the spore is densly packed with granular materials. Even more preferably, the cytoplasm contains several nuclei, in particular about 8 to 25 nuclei (Fig. 4). Typically no clamp connections or sexual structures occur.

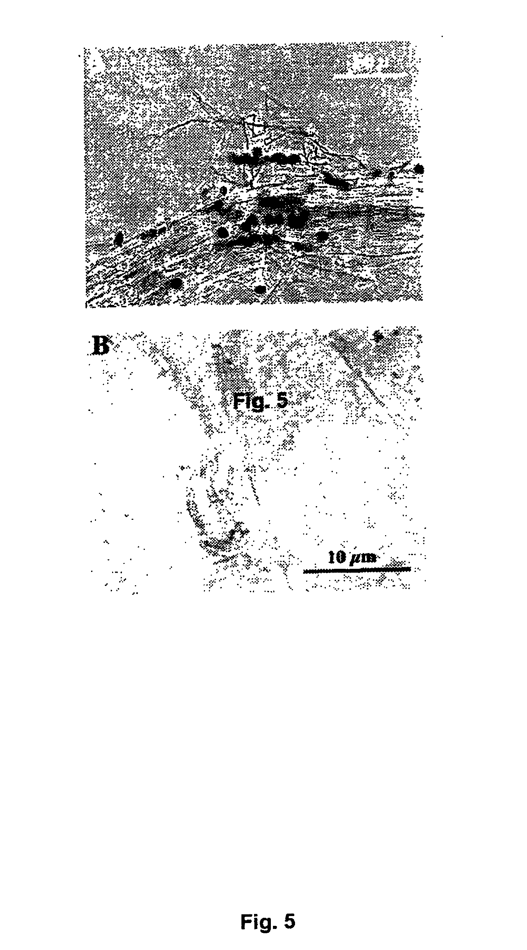

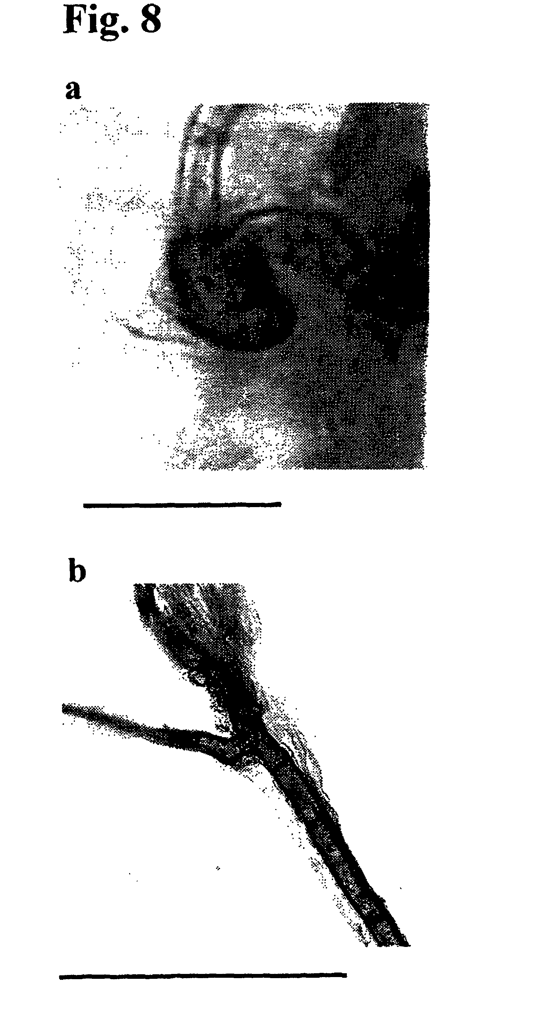

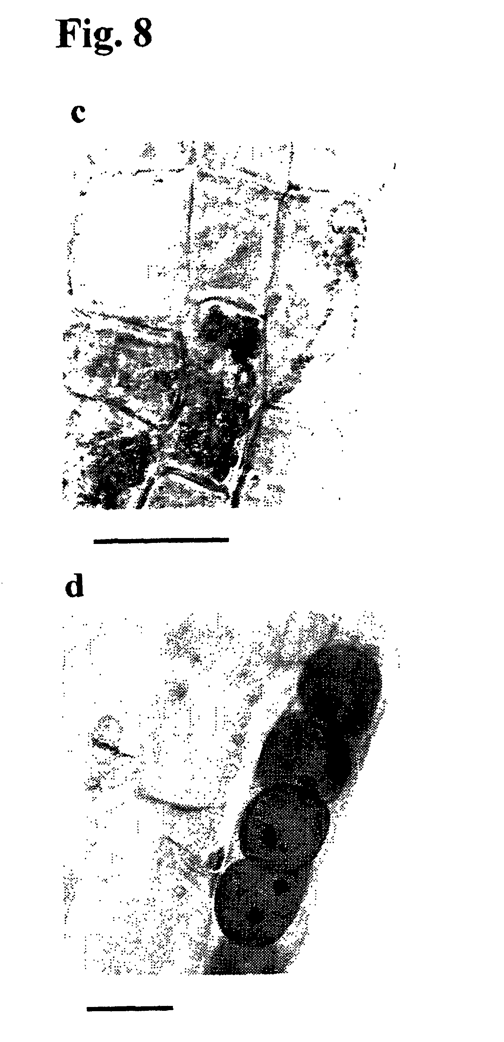

The fungus of the present invention has the capability to interact with the living plant root. This means, that the fungus is not only a saprophyte but is also biotroph. Furthermore, it is preferred that the fungus can live on the root (Fig. 5A) as well as within the root (Fig. 5B). More preferably, the hyphae of the fungus develop appressoria-like structures when they get into contact with living plant roots (Fig. 8a).

In a preferred embodiment the fungus is capable to colonize the living root of plants, preferably the roots of maize or tobacco, both, intercellularly and intracellularly. More preferably, the fungus colonizes the root cortex intra- and intercellularly (Fig. 8b). It is furthermore preferred that the hyphae show further differentiation when growing in the cells. Preferably, this differentiation is the development of intracellular coils or branches (Figure 8c) and spore- or vesicle-like structures (Figure 8d). More preferably, the hyphae of the fungus of the invention do not invade stelar tissue and do not traverse upwards into the shoot when interacting with the living plant.

In a further preferred embodiment, the fungus according to the invention is able to produce chlamydospores by the mycelium growing within the living plant root as well as by the mycelium growing outside of the living plant root.

In another preferred embodiment the chlamydospores produced by the fungus according to the invention measure about 14 to 33 µm in length and 9 to 20 µm in width.

In a further preferred embodiment the fungus according to the invention is capable of growing on various natural and synthetic media. In particular, the fungus can be easily cultivated in or on a multitude of different media and substrates. The medium used for cultivating the fungus can be a liquid medium or a solid medium. The fungus can, for example, be cultivated on a wide range of synthetic and complex media which are generally used for the cultivation of fungi, e.g. on minimal medium (MM1) normally used for in vitro germination of AM fungi with 10% sucrose or glucose as carbon source, on media for Aspergillus sp., e.g. CM and MM2 medium and on Moser b medium. The fungus according to the invention can preferably also be cultivated on natural substrates such as young or mature leaves of hemp (Cannabis sativa L.), young leaves of Cynodon dactylon (L.) Pers., on pollen grains, oat meal (Difco), potato carrot, potato dextrose (Difco), tomato dextrose agar, tomato juice (Difco), Lima bean (Difco) or maize meal (Difco).

In a particularly preferred embodiment the fungus according to the invention is the fungus deposited on October 16, 1997 at the DSM (Deutsche Sammlung von Mikroorganismen und Zellkulturen GmbH), Braunschweig, Germany, under Accession number DSM 11827.

Due to the occurrence of typical dolipores with continuos parenthosomes this fungus belongs to the Hymenomycetes (Basidiomycota). However, it does not resemble any known fungus. Accordingly, a new genus was introduced and, for the characteristic spore structure and the place of its isolation it was named Piriformospora indica.
Sequence analysis of the 18S rDNA of the fungus and comparison with the 18S rDNA sequences from other Basidiomycota revealed that Piriformospora indica is related to the Rhizoctonia group (Ceratobasidiales). The highest similarity was found with Rhizoctonia solani and Thanatephorus praticola.

Furthermore, the present invention also relates to the chlamydospore of the fungus according to the invention. Such a chlamydospore has a typical pear-shaped structure. Preferably, the chlamydospore measures about 14 to 33 µm in length and 9 to 20 µm in width. Preferably, very young spores have one-layered thin, hyaline walls. At maturity, they preferably have at least one of the following features: they are thick, double walled, smooth and/or pale yellow. More preferably, cytoplasm in the spore is densly packed with granular materials. Even more preferably, the cytoplasm contains several nuclei, in particular about 8 to 25 nuclei. Typically no clamp connections or sexual structures occur.

The present invention also relates to a mycelium of the fungus of the present invention. The hyphae of this mycelium are septated and are preferably thin-walled and of a diameter of about 0.7 to about 3.5 mm. They may be intertwined and overlap each other when grown on a solid culture medium.
When cultured on a solid culture medium, the mycelium is preferably mostly flat and submerges into the substratum. In older cultures it can happen that hyphae are irregularly inflated, showing nodose to coralloid shape. When the mycelium grows on root surfaces it is possible that granulated dense bodies can be observed.

Furthermore, the present invention relates to a culture of the fungus according to the invention. As already mentioned above, the fungus can be cultivated on a wide variety of synthetic or natural substrates. For example, the fungus can be cultivated on different kinds of solid culture media, such as MM1 medium, different media for Aspergillus sp., e.g. CM and MM2 medium, or Moser b medium. The fungus can also be grown on various natural sources, such as leaves of various plants, pollen grain, oat meal, potato carrot, potato dextrose or tomato dextrose agar.

In a preferred embodiment the culture medium, in which the fungus according to the invention is grown, is a liquid medium. The medium may be a synthetic or complex medium. Preferably, the medium is a medium for Aspergillus sp., e.g. MM2 or CM medium, or Moser b medium. The liquid culture of the fungus according to the invention can be carried out according to methods known in the art. Preferably, the culture medium is constantly agitated during cultivation, e.g. with about 1 rps. Furthermore, the cultivation temperature lies in the range of about 20 to 30°C.

The present invention also relates to a liquid culture medium obtained from a culture according to the invention which is free or essentially free of the fungus according to the invention. This culture medium can be prepared by first cultivating the fungus according to the invention in a liquid culture medium and then separating the liquid culture medium form the fungus growing therein. The separation can be achieved by different means known to the person skilled in the art, e.g. by centrifugation or filtration. It is possible, for example, to heat the medium with the fungus twice up to about 80°C for 30 min and then remove the fungal material by centrifugation.

In a preferred embodiment the liquid culture medium obtained from a culture of the fungus is a filtrate. Preferably, such a filtrate is obtained by filtration of the culture medium through a filter having a pore size of no more than 2 µm, preferably through a filter having a pore size of no more than 0.2 µm. The filtration step should preferably lead to the removal of essentially all the fungal hyphae, more preferably the filtration should also remove the chlamydospores and even more preferred it should remove all fungal material.

The present invention also relates to a substrate comprising the fungus, the chlamydospore, the mycelium according to the invention or the culture medium or filtrate thereof according to the invention or any possible combination of any of these components. The substrate may be liquid or solid.

In a preferred embodiment the substrate according to the invention can be used as a growth substrate for plants. Examples are solified synthetic or natural media for the cultivation of plants, especially in vitro. Other examples are soil, sand, humus or peat or mixtures of these.

The present invention also relates to compositions comprising the fungus, the chlamydospore, the mycelium, the culture medium or culture filtrate of the invention or any possible combination of any of these components. Preferably, such a composition comprises furthermore one or more agriculturally acceptable carriers, e.g. an appropriate solid or liquid adjuvant.

In a preferred embodiment the composition according to the invention is a fertilizer.

Furthermore, the present invention relates to the use of the fungus, the chlamydospore, the mycelium, the culture medium or filtrate according to the invention, for promoting development, growth, health and/or nutrition of plants. It has been found that inoculation of substrates with the fungus according to the invention, preferably with mycelium and chlamydospores, increases significantly the growth of plants grown on the inoculated substrate. The same effect could be observed when only the culture medium or filtrate of a liquid culture of the fungus was used.

Accordingly, the present invention also relates to a process for promoting plant development, growth, health and/or nutrition which comprises the step of adding the fungus, mycelium, chlamydospore, culture medium or filtrate according to the invention or a combination of any of these components to the substrate on which the plant is grown or to the plant itself or to the substrate and the plant.

The promotion of health preferably means an increased resistance to pathogens. In a preferred embodiment the promotion of plant health is a prophylaxis against damage of the plant's roots by other organisms. In particular, the growth of the fungus according to the invention on living plant roots protects these roots from negative effects caused by some other organisms, in particular by Rhizoctonia spec. or Fusarium spec. The promotion of resistance can be tested, e.g., by first inoculating a plant with the fungus according to the invention followed by an inoculation of the roots of the plant, for example, with Rhizoctonia or Fusarium or of the leaves with Alternaria and then determining the effect of the pathogen.

Furthermore, a promotion of plant health could mean that the fungus, mycelium, chlamydospore, culture medium or filtrate is used to treat a contaminated soil on which plants are grown in order to render the plants more tolerant against toxic substances, such as heavy metals or chlorinated or nitrated hydrocarbons, and/or to prevent that these substances enter the food chain. A promotion of health in this context can be determined, for example, by cultivating plants inoculated with the fungus according to the invention and control plants on substrates contaminated with heavy metals and by comparing the biomass production and heavy metal concentrations.

In another preferred embodiment the promotion of plant growths results in at least one of the following effects:
- an increase in the overall yield of the plant;
- an increase in quality of the plant material;
- an increase in chlorophyll content of photosynthetic tissue;
- an increase of the growth of root; and
- an increase of the overall biomass production of the plant.

An increase in the overall yield means preferably an increase of the yield of harvestable parts of the plant. Depending on the plant these may be for example, the tuber (e.g. potato), the turning (e.g. sugar beet), the stem (e.g. sugar cane), the leaves (e.g. tobacco, Artemisia), the fruit (e.g. tomato, cucumber, pumpkin, grape, apple, peach, cherry etc.) or the seed (e.g. cereals, such as maize, rice, rye, barley, wheat, oat; cotton). The yield may be, e.g., either increased in so far as the number of harvestable parts is increased and/or as their weight and/or content of storage substances, interesting metabolites etc. is increased.

An increase in quality means preferably, an improvement of the desired qualities of a plant. These again, can differ from plant to plant. For example, with respect to ornamental plants, e.g. Petunia, an increase in quality can mean an increase in the number of flowers or the number of leaves. With respect to cereals an increase in quality can mean an increase in protein or starch content in the seeds or a desired alteration in the structure or composition of storage substances.

An increase of the growth of the shoot preferably means an increase in the overall heights of the plant.

An increase in chlorophyll content of photosynthetic tissue means preferably an increase of about at least 10% when comparing untreated with treated plants.

An increase of the growth of roots means preferably that the treated roots show a stronger ramification and a much higher number of root hairs than untreated roots.

An increase of the overall biomass production of the plant means that the biomass of a plant is at least 20%, preferably at least 40%, more preferably 60% and particularly preferred at least 90% higher when treated with the fungus, chlamydospore, mycelium, culture medium or filtrate according to the invention in comparison to untreated plants. More preferably the biomass of treated plants is at least about twice as high as the biomass of untreated plants.

The promotion of development of the plants means for example, that the development of flowers and fruit is accelerated, that the germination of seeds is accelerated or that regeneration of plantlets from cells or callus is accelerated.

In a preferred embodiment the promotion of plant nutrition results in an increase of phosphate uptake.

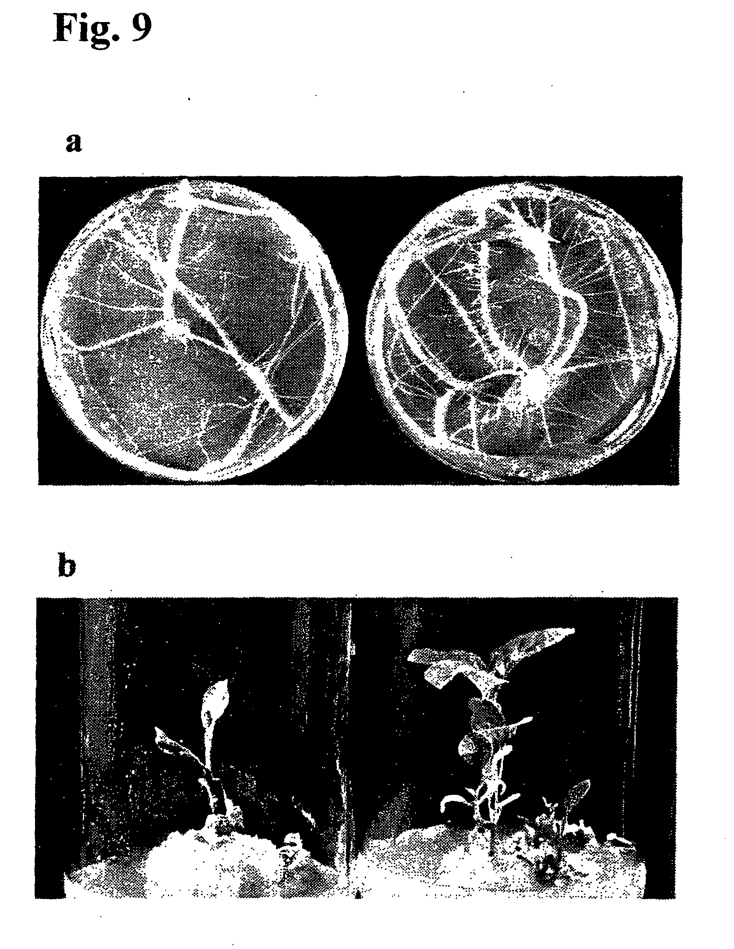

Furthermore, the present invention relates to the use of the fungus, the chlamydospore, the mycelium, the culture medium and/or the culture filtrate according to the invention for rendering possible or promoting the regeneration of plants from plant material, such as plant cells, e.g. protoplast or single cells with cell walls from various tissues, tissue explants, cuttings from stems, leaves or roots or callus tissue. It has been observed that the fungus according to the invention, when applied to callus tissue or to the culture medium on which the callus is cultivated, can induce early differentiation of the callus into roots and shoots (Figure 9b). Furthermore, when regenerated plantlets, which are colonized by the fungus, are transferred to soil they attain early establishment and the survival rate is almost 100%. In some instances, it was found that addition of the fungus makes possible regeneration of plantlets from callus where this has been difficult so far, e.g. for egg plant. In a preferred embodiment the plant cells, cells of the tissue explant or cells of the callus are transformed cells, i.e. cells in which a DNA molecule has been introduced by one of the methods known in the art. Thus, the fungus, chlamydospore, mycelium, culture medium and filtrate according to the invention are particularly useful for the regeneration of plants which could not be regenerated up to now when starting from cells, tissue explants or callus tissue. Furthermore, they greatly improve the overall efficiency of regeneration in so far as they induce early differentiation, enhance the development, increase growth and cell biomass and lead to a highly increased survival rate.

Accordingly, the present invention also relates to a process for the regeneration of plants from plant material which involves the addition of the fungus, the chlamydospore, the mycelium, the culture medium and/or the filtrate according to the invention at least at one stage of the regeneration process to the substrate on which the plant material or the developing plantiet is cultivated and/or to the plant material or the developing plantlet itself.

Furthermore, the present invention relates to the use of the fungus, chlamydospore, mycelium, culture medium and/or filtrate according to the invention for promoting germination of plant seeds. It was found that the addition of at least one of these substances to seeds or to the substrate in or on which they are kept significantly promotes germination of the seeds (Figure 9a). In particular, the seeds germinate earlier, they produce more roots and root hairs, the overall biomass of the developing seedling increases faster and the developing leaves have a higher chlorophyll content.

Thus, the present invention also relates to a process for promoting germination of plant seeds comprising the step of adding the fungus, the mycelium, the chlamydospore, the culture medium or the filtrate according to the invention or a mixture of any of these components to the substrate on or in which the seed germinates or to the seed itself or to the substrate and the seed.

Moreover, the present invention relates to the use of the fungus, the mycelium, the chlamydospore, the culture medium or the filtrate according to the invention or a combination of any of these components for the treatment of a substrate suitable for plant growth in order to improve its structural, chemical composition and/or biological composition for the culture of plants. The treated substrate contains preferably soil, sand, humus, peat or a combination of any of these components. The substrate is preferably treated in a way that the fungus is cultivated in it before the substrate is used for the cultivation of plants.

The above-mentioned uses and processes can be carried out with all possible plants and cells or tissue derived from such plants. In particular, they can be carried out with all Gymnosperms and Angiosperms (monocotyledonous or dicotyledonous) or plant material derived from such plants, including trees, shrubs, herbs and grasses. Preferably, the uses and processes are applied to useful plants or material or cells of such plants, more preferably to agricultural, horticulture or forestry plants. Examples are trees and shrubs, vegetable plants, such as tomato, cucumber, egg plant, zucchini, beet, mustard, carrot, rape, pumpkin, melon, salad, leek onion, different legumes like pea, fababean, Trifolium, Medicago, etc., fruit plants, such as orange, grape apple rasberry, blackberry, strawberry, pear, plum, cherry, etc., cereal plants, such as wheat, barley, rye, oats, maize, rice, millet, or other agriculturally interesting plants such as potato, cassava, sugar beet, sugar cane, cotton, hemp, linseed, flax etc. Plants which are interesting for forestry are, e.g., poplar, eucalypt, spruce, pine etc. Plants interesting for horticulture are, for example, Petunia, Begonia, Geranium, Chrysanthemum. Furthermore, plants for medical uses, like Artemisia, Bacopa etc. can be used.
- **Figure 1**: shows colony growth of *Piriformospora indica.* An agar disk was transferred onto CM medium and incubated at 30°C. Pictures were taken (A) 15 days and (B) 30 days after inoculation.
- **Figure 2**: shows the morphology of hyphae. *P. indica* was grown on (A) MM agar, on (B) Moser B agar, on (C) the surface of a maize root and on (D) MM2 agar. Hyphae were photographed under the light microscope without staining (A, B), after trypan blue staining (C) or under the epifluorescense microscope after calcofluor staining.
- **Figure 3**: shows chlamydospores of *Piriformospora indica.*
- **Figure 4**: shows nuclei in a chlamydospore. Chlamydospores were stained with DAPI and observed by epifluorescense microscopy. Different optical plains were assembled in one picture using the Improvision software package (IMPROVISION, Coventry, UK).
- **Figure 5**: shows root colonization. Maize roots were grown with *P. indica* and harvested (A) 7 days or (B) 14 days after inoculation. The fungus was stained with trypan blue (A) or chlorazol black E (B).
- **Figure 6**: shows dolipore and parenthosomes of *P. indica.* Cuttings of hyphae were observed by TEM. Cell wall (1) and parenthosomes (2) are indicated by arrows.
- **Figure 7**: shows a dendrogram of evolutionary distances based on part of the 18S rDNA sequences of different fungi. The comparison was carried out based on the Jukes-Cantor equation. Numbers indicate the significance of a boots trap analysis. The bar indicates 2% in nucleotide differences.
- **Figure 8**: shows plant colonization and infection by *Piriformospora indica.* The scale bars below all photos indicate a length of 10 µm. **a**, A fungal appressorium stained with trypan blue attached to the root of maize (Zea *mays* L.). **b**, Light microscopy of a maize root 7 days after inoculation with the endophyte. The trypan blue stained segment shows intercellular hyphae. **c**, Light microscopy of a maize root 16 days after inoculation showing coiled (arrow 1) and branched (arrows 2) intracellular hyphae. **d**, The same maize root with intracellular clusters of round bodies.
- **Figure 9**: shows plant development enhancing effect of *Piriformospora indica.* **a**, Maize seed germination on minimal agar medium containing 10% sucrose. Seedling on the right was treated with two agar disks (1 cm in diameter) containing hyphae and a few chlamydospores of the fungus (right plant) or control agar disks without the fungus (left plant). One surface sterile seed was placed on the agar plate. Arrows indicate two fungal agar discs each 1 cm in diameter. Incubation was allowed for 14 days. **b**, Calli of egg plants (*Solanum melangena*) differentiate into shoot and leaves within 14 days of incubation on treatment of the callus tissues with the fungus (as under **a**) and left served as the control.

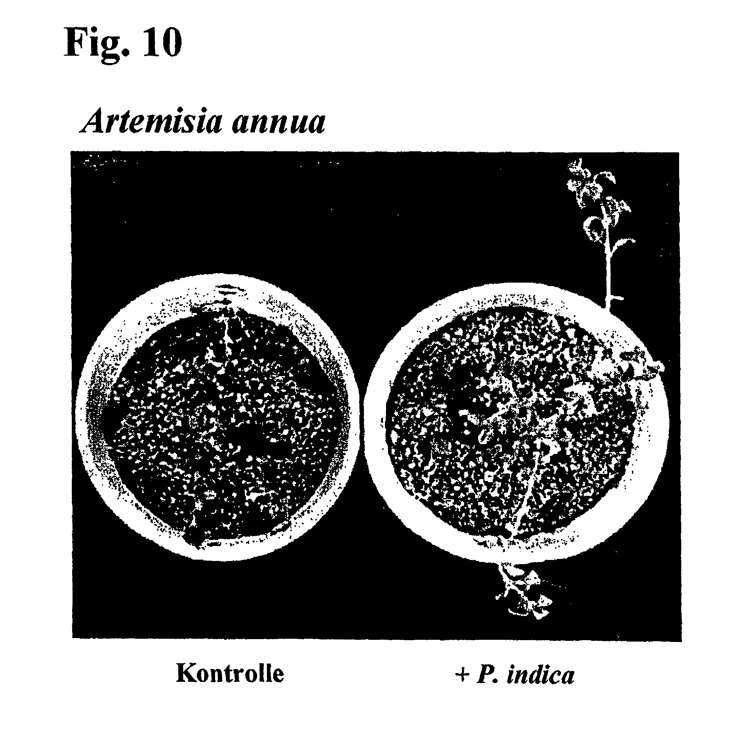

- **Figure 10**: shows a comparison of Artemisia annua plants cultivated in the presence of P. indica (right plant) and in the absence of P. indica (left plant). Plantlings were grown from calli as described in Varma (Crit. Rev. Biotech. 15 (1995), 179-199). When transferring the plants to pots they were inoculated with the fungus as described in Example 2. The Figure shows plants 4 weeks after inoculation.

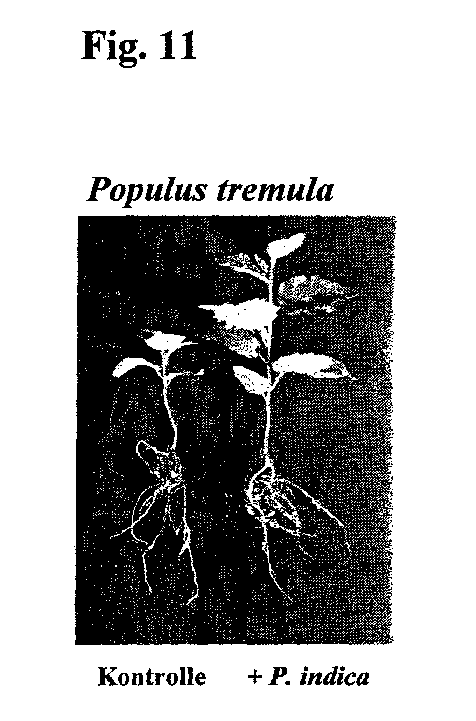

- **Figure 11**: shows a comparison of Populus tremula plants cultivated in the presence of P. indica (right plant) and in the absence of P. indica (left plant). The plants were treated as described for Figure 10.
- **Figure 12**: shows maize plants treated with the culture filtrate (CF) of Piriformospora indica (right) and untreated control plants (left).
- **Figure 13**: shows the biomass of leaves of maize plants treated with the culture filtrate (CF) of Piriformospora indica (right) and of untreated control plants (left).

The following Examples further illustrate the invention:

### Example 1

### Isolation of a new fungus named Piriformospora indica

Field-isolated spores of arbuscular mycorrhizal (AM) fungi are often contaminated not only with bacteria, but also with fungi (Lee, Mycol. Res. 98 (1994), 458-466) some of which can act as hyperparasites (Ross, in: Methods and principles of mycorrhizal research, Eds. Schenk, American Pathological Society, St. Paul, USA (1982), 55-58). A new fungus appeared during routine monosporic mass inoculum preparation of *Glomus mosseae* isolated from a desert soil in north-west of India.

**Isolation and cultivation:** Arbuscular mycorrhizal (AM) fungal spores were obtained from the rhizosphere soils of woody shrubs *Prosopis juliflora* (Swartz) Dc. and *Zizyphus nummularia* (Burm. f.) Wt. & Arn. in the sand dunes desert soils of Rajasthan, north western India. Establishment of single-species pot cultures from arbuscular mycorrhizal fungal spores were carried out by placement of single newly formed spores near the roots of 5-7 d-old maize seedlings as described (Varma, Crit. Rev. Biotechnol. 15 (1995), 179-199). Trap cultures were harvested 3 months after inoculation and spores were isolated (Gerdemann, Trans. Brit. Mycol. Soc. 46 (1963), 235-244), surface sterilized and placed on minimal medium (MM1, Williams in: Methods in Microbiology, Eds. Norris and Read, Academic Press, London (1992), 201-209). Hyphae which grew out and which were obviously not from an AM fungus were excised and transferred to a fresh medium. After several transfers they achieved the status of an axenic culture (Lane, Can. J. Bot. 41 (1974), 1-17).
At a later stage, the new fungus was also grown on agar plates and in liquid culture using several other media containing complex nutrients, namely, CM (complex medium for *Aspergillus,* Pontecorvo, Adv. Genet. 5 (1953), 141-238), MM2 (minimal medium for *Aspergillus,* Käfer, Adv. Genet. 19 (1977), 33-131) and Moser B medium (Moser, "Die Gattung Phlegmacium", Klinkhardt, Bad Heilbrunn, Austria). Germlings and chlamydospores were fixed in 8 % formaldehyde in PME buffer (50 mM PIPES, pH 6.9; 25 mM EGTA; 5 mM MgSO₄) for 45 min, washed with PME buffer three times and then mounted in Vectashield media with DAPI (1.5 µg/ml; Vector Laboratories, Burlingame, CA, USA) for the observation of nuclei and with calcofluor (1.5 µg/ml) for the visualization of septa (Fischer, J. Cell Biol. 128 (1995), 485-498).

### Example 2

### Characterization of the fungus

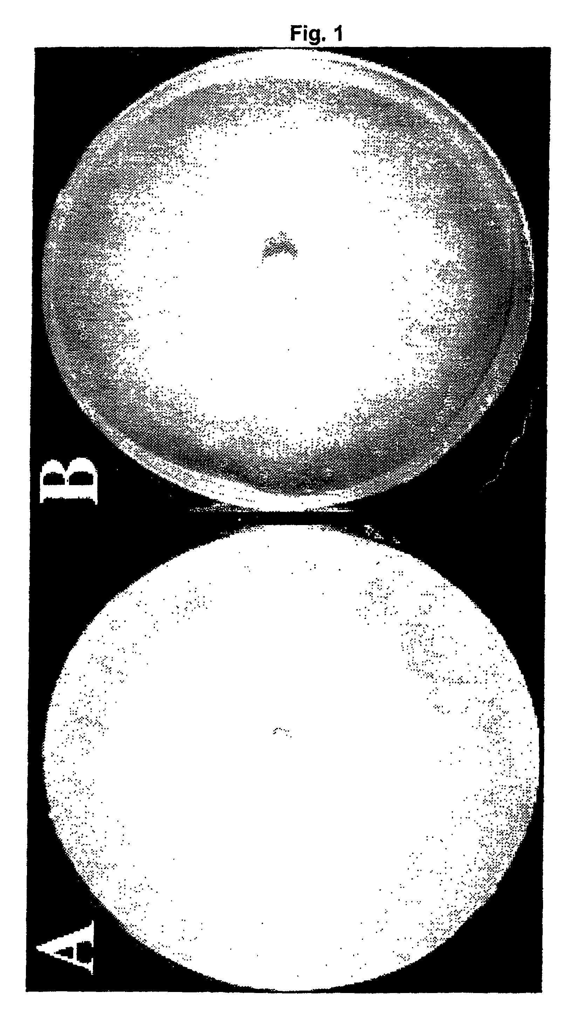

**Morphology:** The isolated fungus grew on a wide range of synthetic and complex media, e.g. on minimal medium (MM1) normally used for *in vitro* germination of AM fungi with 10% sucrose or glucose as carbon source, on two different media for *Aspergillus* sp. (CM and MM2), and on Moser b medium. Young mycelia were white and almost hyaline (Fig. 1a), but inconspicuous zonations were recorded in older cultures (Fig. 1b). The mycelium was mostly flat and submerged into the substratum. Hyphae were thin-walled and of different diameter ranging from 0.7 to 3.5 mm (Fig. 2a). They often intertwinded and overlapped each other (Fig. 2b). In older cultures, hyphae were often irregulary inflated, showing a nodose to coralloid shape (Fig. 2c, Fig. 3). Granulated dense bodies were mainly observed when the fungus grew on root surfaces (Fig. 2c). The septated hyphae showed sometimes anastomosis (Fig. 2d). Since septation was irregular, the single compartments could contain more than one nucleus. Chlamydospores were formed from thin-walled vesicles at the tips of the hyphae (Fig. 3). They appeared singly, or in clusters. These chlamydospores were distinctive due to their pear shaped structure measuring (14)16-25(33) µm in length and (9)10-17(20) µm in width. Very young spores have one-layered thin, hyaline walls. At maturity, they appeared thick (-1.5 µm), double walled, smooth and pale yellow. The cytoplasm was densely packed with granular materials and usually contained 8-25 nuclei (Fig. 4). Neither clamp connections nor sexual structure could be observed.
Significant quantitative and morphological changes have been detected when the fungus was challenged to grow on different media: the incubation in a shaking state retarded the growth in MM1 liquid cultures (12-7 g fresh weight/L after 2 weeks at 30°C), whereas no such negative effect was ever observed during cultivation in any other substrate. Intact, the fungal biomass was considerably enhanced on shaking cultures with *Aspergillus* CM (sometimes up to 50 g fresh weight/L after 2 weeks at 30°C). On Moser b medium, the colonies appeared compact, wrinkled with furrows and constricted. The mycelium produced defined zonations and high amount of white aerial hyphae. Hyphae were highly interwoven, often adhered together and gave the appearance of simple cords. New branches emerged irregularly and, the external wall of hyphae at regular intervals showed some deposits, perhaps polysaccharides and/or some hydrophobic proteins, deeply stained with toluidine blue. Chlamydospores were very sticky (almost glued) and it required drastic and vigorous physical treatments to segregate them. The submerged mycelium produced on MM1, CM and MM2 also produced chlamydospores with low to high frequency, but the chlamydospores were scanty and often in loose clusters.

The mycelium, i.e. the hyphae and the spores of the fungus, were further examined by transmission electron microscopy. For this purpose, mycelium and chlamydospores from liquid cultures were fixed overnight with 2% glutaraldehyde in 0.1 M sodium cacodylate buffer (pH 7.2) at room temperature. Following six transfers in 0.1 M sodium cacodylate buffer, samples were postfixed in 1% osmium tetroxide in the same buffer for 2 h in the dark, washed in distilled water, and stained in 1% uranyl acetate for 1 h in the dark. After three washes in distilled water, samples were dehydrated in acetone using 10 min changes at 10%, 25%, 50%, 70%, 95% acetone and three 10 min changes in 100% acetone. Samples were embedded in Spurr's plastic (Spurr, J. Ultrastruct. Res. 26 (1969), 31-43).
Sections were cut using a Reichert-Jung Ultracut E equipped with a diamond knife and mounted on Formvar-coated single-slot copper grids. The sections were stained with lead citrate (Reynolds, J. Cell. Biol. 17 (1963), 208-212) at room temperature for 5-8 min and washed with distilled water. They were examined with a Phillips 301 G transmission electron microscope.

**Ultrastructure:** In order to get more information about the systematic position of the new fungus, the ultrastructure of the septal pore and the cell wall were examined. The cell walls were very thin and showed multilayerd structures (Fig. 6, arrow 1). The septal pores consisted of dolipores with continuous parenthosomes (Fig. 6, arrow 2), which proofs the systematic position within the Hymenomycetes. The dolipores were very prominent with a multilayered crosswall and a median swelling mainly consisting of electron transparent material. The electron transparent layers of the cross walls extend deep into the median swellings but do not fan out.
In median sections of the septal pores, the parenthosomes were always straight and had the same diameter as the corresponding dolipore. Any kind of pores could not be detected. This means, that they are flat discs without any perforation. The parenthosomes consisted of an electron dense outer layer and a less dense inner layer, which showed an inconspicuous dark line in the median region. They were in contact with the ER and membranes were mostly found near the dolipore.

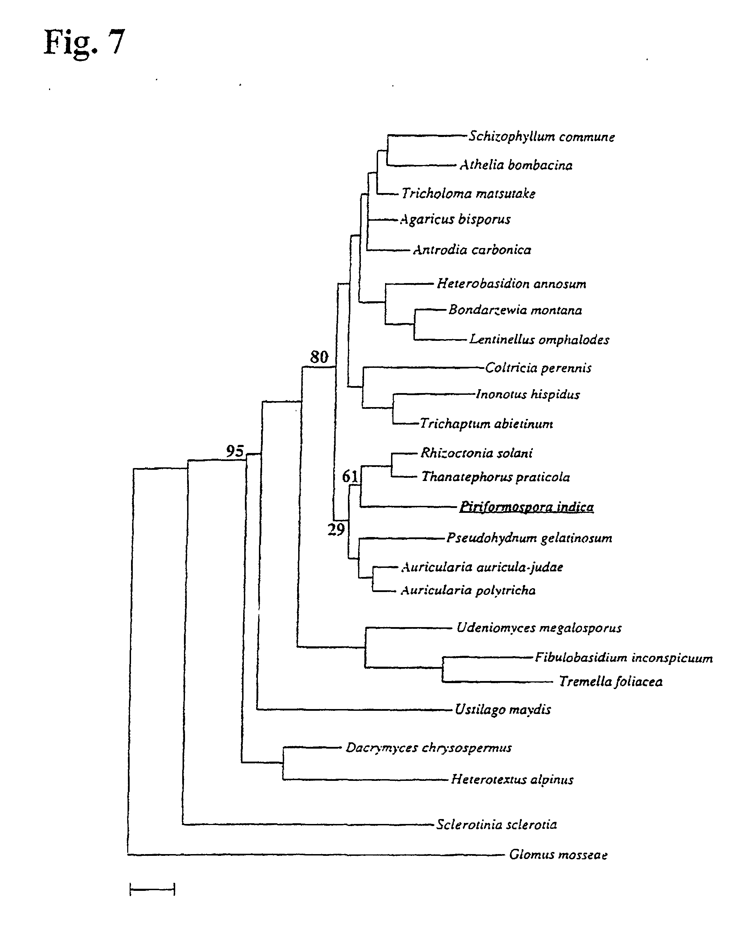

**Analysis of 18S rDNA:** Furthermore, in order to get a more precise idea of the closest relatives of the isolated fungi, 18S rDNA was isolated and analyzed. For this purpose, young mycelium harvested from liquid CM cultures was used for DNA extraction following the method for filamentous fungi of Timberlake (Science 244, (1989), 1313-1317). PCR was conducted with 100 ng of genomic DNA using the primer pair NS1 and NS2 (White, in PCR protocols, Eds Innis, Gelfand, Suinsky, White, Academic Press, N. Y. (1990), 315-322). The amplification was carried out in 20 µl volume with 0.5 units *Taq*-polymerase (Gibco-BRL), 200 µM dNTPs and 1µM of each primer under buffer conditions as recommended from the supplier of the enzyme. First denaturing was conducted at 95°C for 5 min followed by 30 cycles with each step for 1 min (94°C; 50°C; 72°C) and a final elongation at 72°C for 5 min. PCR products were cut from 2% agarose gels, purified using the Geneclean system (BIO101, Dianova) and cloned into the pT7Blue vector according to the supplier (Novagene). Recombinant plasmids were used to transform electro-competent *E. coli* XL1-Blue cells (Sambrook, Molecular Cloning: A Laboratory Manual, Cold Spring Harbor Laboratory Press, Cold Spring Harbor, N. Y. (1989)). PCR clones derived from three independent cultures were grown, plasmid DNA was isolated on anion exchange resin columns (Genomed) and sequenced on the sequencing automate Alf Express (Pharmacia) using the Cy5™-labeled (Biological Detection Systems) universal and reverse primers 18.1 and 18.2. The sequence appears in the EMBL/Genebank under the accession number AF014929 (5' region of the 18S rRNA). Similarities were calculated by comparing 525 nucleotide positions. Evolutionary distances between the organisms have been estimated by the Jukes-Cantor equation (Jukes, in: Mammalian protein metabolism, Eds. Munro, Academic Press, N.Y. (1969), 21-132) using the DNANIST program of the PHYLIP package (Felsenstein, PHYLIP (Phylogeny Inference Package) Version 3.5p, University of Washington, Seattle). In order to test the statistical significance of the derived dendrogram, a bootstrap analysis of 500 data resampling using the neighboring-joining method of the MEGA program (Kumar, MEGA: molecular evolutionary genetic analysis, Version 1.0, Pennsylvania State University) was carried out.
According to the above-described methods part of the 18S rRNA was amplified, sequenced and compared with all corresponding data of different Basidiomycota available in the Genebank sequence library, as well as of some Ascomycota and Basidiomycota (data not shown). Based on these results a second analysis was carried out using only two outgroups (one Ascomycete and one Zygomycete) and only those Basidiomycota which contribute to the understanding of the evolutionary relationships of the new fungus (Fig. 7). The dendrogram mirrored the classification within the Basidiomycota into the different orders with the exception of the Aphyllophorales and the Agaricales which were grouped together. The closest similarity of the 18S rRNA sequence of the new fungus appeared to the *Rhizoctonia* group (Ceratobasidiales), namely *Rhizoctonia solani* and *Thanatephorus praticola. P. indica* has with those a common ancestor with a boots trap value of 61%. When. the same analysis is carried out without this group, the new fungus does not match up with any other species.

**Infection of plant roots:** Maize (*Zea mays* L.) seedlings were grown from surface-sterilized seeds on expanded clay under conditions as described for parsley (Franken, Mol. Plant-Microbe Interact. 7 (1994), 612-620). On transfer of the seedlings into pots, 5g (fresh wt) of inoculum containing mycelium + chlamydospores harvested from liquid CM cultures were mixed with the substrate and placed about 1 cm below the surface. Plants were harvested at different time points after inoculation, roots were stained with chlorazole black E (Brundrett, Can. J. Bot. 62 (1984), 2128-2134) or trypan blue (Dickson, in: Mycorrhizal Manual, Eds. Varma, Springer Verlag, Heidelberg, Tokyo, N. Y. (1997), 77-84) and examined by light microscopy.
In particular, pot cultures of maize were inoculated with the mycelium of the fungus in order to examine its ability to interact with plant roots. First infection structures like appressoria were observed after 7 days (Fig. 5a). One week later, chlamydospores developed outside (Fig. 5b) and within (Fig. 5c) the roots.

**Conclusion:** The classical species concept is mostly based on morphology. In systematics, individuals are grouped on the basis of similarities and are distinguished from others on the basis of discontinuities in their characters. This concept has been used more or less successfully, but it is difficult to apply to asexually reproducing fungi. The new fungus only produces chlamydospores at the apex of undifferentiated hyphae. Different kinds of substrates were tested to induce sexual development, like young and mature leaves of hemp (*Cannabis sativa* L.), young leaves of *Cynodon dactylon* (L.) Pers. and pollen grains, oat meal, potato carrot, potato dextrose or tomato dextrose agar. Since all these efforts did not lead to desirable results, there were only a few features to characterize the fungus morphologically and to group it according to the classical species concept.
Investigations on the ultrastructure of the mycelium showed multilayered cell walls and dolipores with continuous parenthosomes. This proved, that the fungus belongs to the Hymenomycetes (Basidiomycota). Dolipores with continuous parenthosomes occur only among the Hymenomycetes and are restricted to a few orders (Auriculariales, Dacrymycetales, Hymenochaetales, Tuiasnellales) and genera (*Botryobasidium*, *Clavulicium*, *Hyphodontia*, *Paulicorticium. Trichaptum*). Some of them have also anamorphic stages, especially some species of the genus *Botryobasidium*, which form anamorphs belonging to the genera *Haplotrichum* and the monotypic *Allescheriella* (Langer, J. Cramer, Berlin (1994)). Among those, only the characters of *A. crocea* (Mont.) Hughes, which is the anamorph of *Botryobasidium croceum* Lentz (Lentz, Mycopathol. Mycol. Appl. 32 (1967), 1-25), show some similarities to the structures found in the new fungus. Both form thick-walled, ± pigmented spores with two, in the LM distinguishable wall layers (Hughes, Mycol. Pap. 41 (1951), 1-17). The size and shape of the spores are also similar. The hyphae of *B. croceum* are, however, constantly broader than those of the new fungus and they show a very characteristic mode of branching, as in all species of the genus *Botryobasidium,* which is different from that of the endophyte. Unfortunately, no culture characters of *A. crocea* are available, so the comparability of the features discussed remains questionable. *A.* crocea is, however, only reported from rotten wood and expected to be saprotrophic, as it is typical for *Botryobasidium* species. In contrast, the new fungus was isolated from spores of *Glomus mosseae* and interacted with roots of living plants. Furthermore, all species of the genus *Botryobasidium* so far studied according to their septal pores (Langer, loc. cit.) had parenthosomes which form cup-like structures covering the median swellings while the new fungus shows disk-like structures never reaching beyond those.
In the last years more and more molecular systematics are used for the classification of fungi. The broadest range over the different levels of systematics is achieved by sequencing of the nuclear rDNA genes (Bruns, Ann. Rev. Ecol. Syst. 22 (1991), 525-564). Therefore, it was decided to analyze the 5' part of the 18S rRNA where most sequences are available in the database. The results showed clearly that the new fungus belongs to the Basidiomycota. The molecular analysis indicated a relatively close relationship to the *Rhizoctonia* group, but the probability for a common anchestor is with a boots trap value of 61% not very significant. Unfortunately, member of the Tulasnellales or of the genus *Botryobasidium* were up to now not analyzed by molecular methods. Members of the Ceratobasidiales are well studied according to their septal pores. All genera of that order have a very characteristic type of dolipores with discontinuous parenthosomes and the median swelling shows a feathery structure (Tu, "Culture, development and sexual states of Rhizoctonia, Sclerotium and related fungi", PhD thesis (1974), University of Florida, Gainsville, USA; Tu, Bot. Gaz. 139 (1978), 454-466; Langer, loc. cit.). No chlamydospore production is known from the members of Ceratobasidiales, but several species are known to produce sclerotia as anamorphic structures belonging to the form genus *Rhizoctonia.* Interestingly, *Rhizoctonia* species are also able to interact with plants, but mostly as root pathogens (Domsch, Compedium of soil fungi, Academic Press, London (1980)) or with orchids as mutualists (Smith, Mycorrhizal symbiosis, Academic Press, London (1997)).
In summary, there is no existing genus which covers all the characters of the new fungus. *Allescheriella,* as a monotypic genus with some similarities according to the chlamydospores, is different because it's a saprotrophic wood decomposer with characteristic *Botryobasidium-*like morphology of the mycelium and septal pore. Other groups of Basidiomycota which may be related according to the molecular or ultrastructural data are not known to be able to produce chlamydospores or have a different way of life. Therefore, a new genus was erected and the fungus was called *Piriformospora indica.*

### Example 3

### Interaction of the fungus with plant roots and its effect on plant growth and development

Tobacco and maize seedlings were inoculated with Piriformospora indica. The interaction of the fungus with the plants was investigated by microscopy. Results of these investigations are shown in Figure 8. When the hyphae got into contact with roots, they developed appressoria-like structure (Fig. 8a). Furtheron, the fungus colonized the root cortex both inter- (Fig. 8b) and intracellularly. In the cells, the hyphae showed, like AMF, further differentiation. In particular, the fungus developed intracellular coils or branches (Fig 8c) and spore- or vesicle-like structures (Fig. 8d). The hyphae neither invaded the stelar tissue nor traversed upwards into the shoot. inoculation of substrates with *P. indica* (mycelia + chlamydospores) increased the growth of the tested plants, tobacco, parsley, maize, populus and two medicinal hosts, *Artemisia annua* and *Bacopa monnieri* (Figure 9a, Table 1). In general, root and shoot biomass was about twice as much for the treatment with the endophyte compared to controls. In addition, the development of flowers and fruits was accelerated. Treatment of tissue culture raised plantlets (*Solanum melongena, Artemisia annua* and *Bacopa monnieri*) with the fungus not only improved growth and higher cell biomass but also induced early differentiation of the calli into roots and shoots (Fig. 9b). Fungus colonized plantlets on transfer to pots attained early establishment and the survival rate was almost 100%. In contrast, the untreated plantlets took almost three weeks longer time for overcoming the "transfer shock" and the survival rate ranged from 30% to 70%. Experiments with maize or parsley seed revealed also a beneficial effect on development. Germination was enhanced and the growth of the plants improved (Fig 9c). In particular, shoots were more chlorophyllous and roots showed a stronger ramification and a much higher number of root hairs.
A lot of research about root-colonizing endophytes with plant growth-promoting effect concerns the AMF, which occur on more than 80% of all land plants. Since they are obligate symbionts, investigations to understand the basis of this interaction and inoculum production for their application are complicated and time consuming. In contrast, ericoid mycorrhiza or a number of ectomycorrhiza can be grown in pure culture, but their host spectrum is restricted to the Ericaceae or to woody plants. The knowledge concerning other cultivable root endophytes is low. Most of the studies were carried out on fungi in plants of alpine and subalpine regions which were later also detected in other ecosystems. A positive influence on plant growth could be shown, but seemed to be species-specific (Haselwandter, Oecologia 53 (1982), 352-354). In some instances, also other fungi were studied, but those reports concerned only the effect on one plant and/or the enhancement of plant growth was relatively low. *P*. *indica* combines the clear plant growth-promoting effect and the broad host specificity of the AMF with the ability of other fungi to be grown in axenic cultures. It is thus an ideal tool to improve commercial plant production in pots, on the field or in micropropagation and could be very useful for different agro-forestry and flori-horticulture applications.

The following Table illustrates the plant growth promoting effect of P. indica on various plant species.

**Table 1**

| Plant | shoot (weight ratio) | root (weight ratio) |
|---|---|---|
| Populus | 1.9 | 1.9 |
| Tobacco | 1.6 | 1.7 |
| Bacopa | 2.9 | 3.4 |
| Artemisia | 1.7 | 2.5 |
| Maize | 2 | 1.6 |
| Parsley | 1.7 | 1.7 |

Plants were inoculated with 1 g mycelium including chlamydospores per 100 ml substrate. Roots and shoots were harvested after 4 weeks for measuring their weight. The numbers which are shown in the table present the ratio of values obtained from the treatment (endophyte) divided by the values of the control (only medium). Similar results were obtained for pea (Pisum sativum L.) (inoculation, culture filtrate), Arabidopsis thaliana L. (inoculation, culture filtrate) and tomato (Lycopersicum esculentum L.). Furthermore, plant growth promoting effects were observed by micropropagation and acclimatization for tobacco (Nicotiana tabacum L.)

### Example 4

### Effect of the culture filtrate on plant growth and development

In order to investigate whether also the culture medium in which the newly discovered fungus was cultured has on its own an effect on plant growth and development similar to that of the fungus, the fungus was cultured in MM2 liquid medium for 21 days and the medium was filtrated through a filter with a pore size of 0.2 µm.

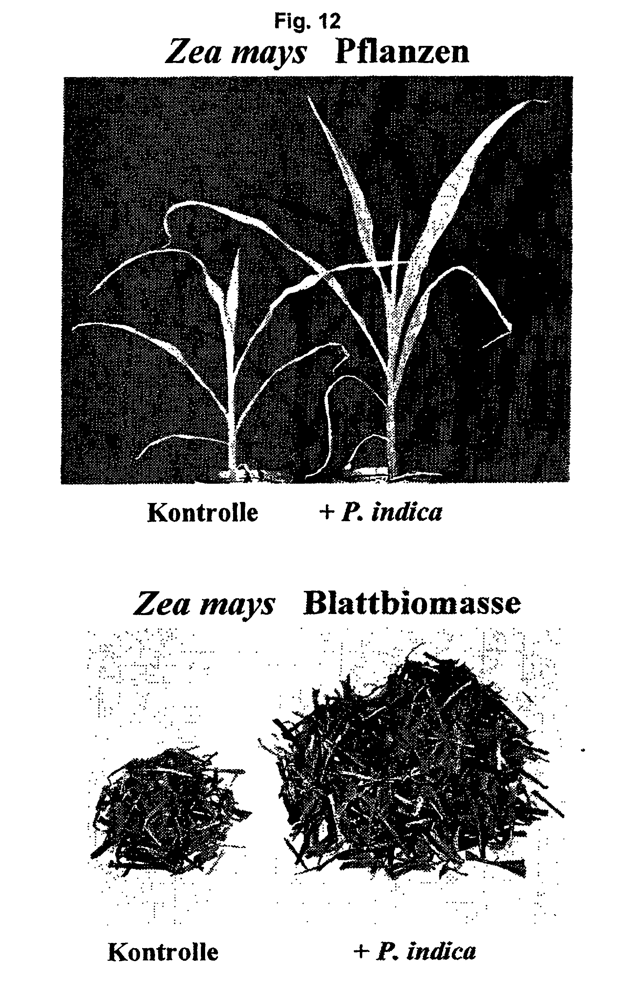

Surface-sterilized maize seeds were germinated on water agar and planted into pots with 250 ml expanded clay. Among normal fertilization, each week 200 µl of the culture filtrate or of the growth medium (control) was added to the pots. The plants were harvested 4 weeks later and the dry biomass was determined. The plants treated with the culture filtrate developed faster than untreated plants (Fig. 12). The shoot weight ratio (cf/control) was in the average 2.3 while the root weight ratio was in the average 1.1. Furthermore, the leaf biomass of plants treated with the fungus was extremely increased (Figure 13). Similar results were obtained with tobacco and parsley.

### Example 5

### Effect of the fungus on phosphate uptake of plants

Parsley plants were grown and inoculated as described with the exception that in one series of experiments the fertilizer was depleted of phosphate. 4 weeks after inoculation they were harvested, dried in an oven (5h; 500°C) and phosphate content was determined by the Molybdo-P blue method as described by Herbert et al. (In: Norris, Ribbons (eds) Methods in Microbiology 5b, Academic Press, New York (1971) 209-334). The results of these experiments are shown in Table 2. As is evident from this Table, the inoculation with the fungus leads to an increase of phosphate uptake by the plants. In principle there are two possibilities to explain the results. The fungus could take up the phosphate in the soil and transport it via the hyphae into the plant root. On the other hand, the fungus could be able to solubilize the phosphate in the soil with extracellular enzymes making it more available for the uptake systems of the plant.

**Table 2**

| Biomass and Phosphate content of parsley plants: | | |
|---|---|---|
| Sample | weight (g) | P (mg/g) |
| +P,-F root | 0.032 | 2.932 |
| +P,+F root | 0.065 | 3.623 |
| +P,-F shoot | 0.045 | 1.845 |
| +P,+F shoot | 0.091 | 2.732 |
| -P,-F root | 0.008 | 2.134 |
| -P,+F root | 0.032 | 3.243 |
| -P,-F shoot | 0.019 | 1.154 |
| -P,+F shoot | 0.048 | 1.685 |
| +/- P = +/- Phosphate in the Long Ashton fertilizer (Hewitt, Tech. Comm. 22 (1966), 430-434) | | |
| + F = addition of fungal inoculum (as for Table 1) | | |
| - F = addition of growth medium alone (as for Table 1) | | |

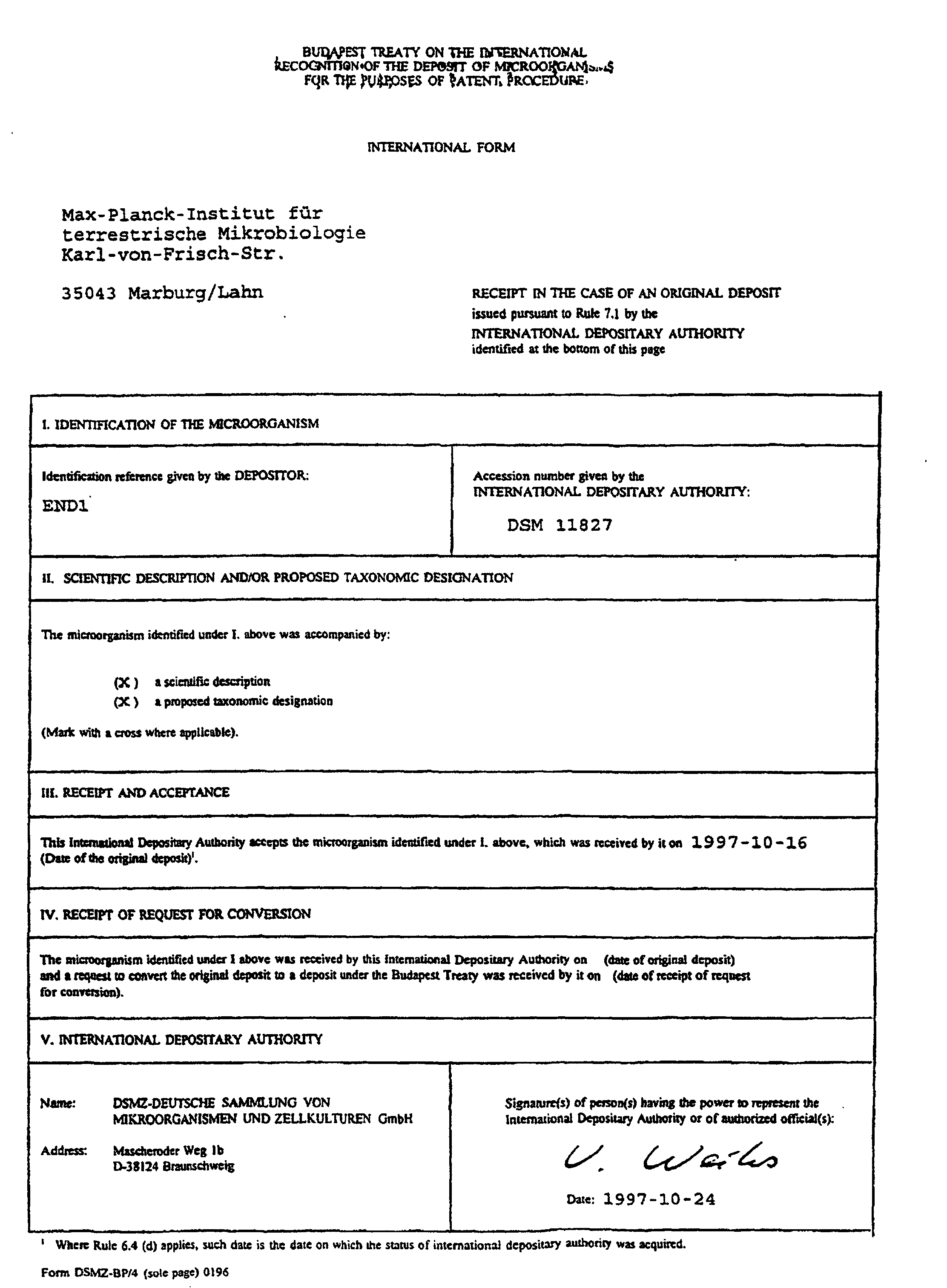
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Max-Planck-Institut für
terrestrische Mikrobiologie
Karl-von-Frisch-Str.

35043 Marburg/Lahn

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the DEPOSITOR:
END1 | Accession number given by the
INTERNATIONAL DEPOSITARY AUTHORITY:
DSM 11827

II. SCIENTIFIC DESCRIPTION AND/OR PROPOSED TAXONOMIC DESIGNATION

The microorganism identified under I. above was accompanied by:

(X) a scientific description
(X) a proposed taxonomic designation

(Mark with a cross where applicable).

III. RECEIPT AND ACCEPTANCE

This International Depositary Authority accepts the microorganism identified under I. above, which was received by it on 1997-10-16
(Date of the original deposit)¹.

IV. RECEIPT OF REQUEST FOR CONVERSION

The microorganism identified under I above was received by this International Depositary Authority on (date of original deposit)
and a request to convert the original deposit to a deposit under the Budapest Treaty was received by it on (date of receipt of request
for conversion).

V. INTERNATIONAL DEPOSITARY AUTHORITY

Name: DSMZ-DEUTSCHE SAMMLUNG VON
MIKROORGANISMEN UND ZELLKULTUREN GmbH

Address: Mascheroder Weg 1b
D-38124 Braunschweig | Signature(s) of person(s) having the power to represent the
International Depositary Authority or of authorized official(s):
Date: 1997-10-24

¹ Where Rule 6.4 (d) applies, such date is the date on which the status of international depositary authority was acquired.

Form DSMZ-BP/4 (sole page) 0196

## Claims

1. A fungus of the genus Piriformospora having at least the following features:
(a) the hyphae are septated;
(b) the septal pores consist of dolipores with continuous parenthosomes;
(c) the mycelium produces at the tips of the hyphae chlamydospores which emerge apically;
(d) the chlamydospores have a pear shaped structure; and
(e) the fungus has the ability to interact with living plant roots.

2. The fungus of claim 1 which colonizes the living plant root both, intracellularly and extracellularly.

3. The fungus of claim 1 or 2, **characterized in that** the chlamydospores are produced by mycelium growing within the living plant root as well as by mycelium growing outside of the living plant root.

4. The fungus of any one of claims 1 to 3 **characterized in that** the chlamydospores measure about 14 to 33 µm in length and about 9 to 20 µm in width.

5. The fungus of any one of claims 1 to 4 **characterized in that** it is capable of growing on various natural and synthetic media.

6. The fungus of any one of claims 1 to 5 which is DSM 11827.

7. A chlamydospore of the fungus of any one of claims 1 to 6.

8. A mycelium of the fungus of any one of claims 1 to 6.

9. A culture of the fungus of any one of claims 1 to 6.

10. The culture of claim 9 which is a liquid culture.

11. A culture medium obtained from the culture of claim 10, which is free or essentially free of fungus.

12. The culture medium of claim 11, which is a culture filtrate.

13. A substrate comprising the fungus of any one of claims 1 to 6, the chlamydospore of claim 7, the mycelium of claim 8, the culture medium of claim 11 or the filtrate of claim 12 or a combination of any of these components.

14. The substrate of claim 13, which is a liquid substrate.

15. The substrate of claim 13, which is a solid substrate.

16. The substrate of any one of claims 13 to 15 which can be used as a growth substrate for plants.

17. A composition comprising the fungus of any one of claims 1 to 6, the chlamydospore of claim 7, the mycelium of claim 8, the culture medium of claim 11 or the filtrate of claim 12 or a combination of any of these components.

18. The composition of claim 17, which is a fertilizer.

19. Use of the fungus of any one of claims 1 to 6, the chlamydospore of claim 7, the mycelium of claim 8, the culture medium of claim 11 and/or the filtrate of claim 12 for promoting development, growth, health and/or nutrition of plants.

20. A process for promoting development, growth, health and/or nutrition of plants comprising the step of adding the fungus of any one of claims 1 to 6, the chlamydospore of claim 7, the mycelium of claim 8, the culture medium of claim 11 or the filtrate of claim 12 or a combination of any of these components to the substrate on which the plant is cultivated or to the plant itself or to the substrate and the plant.

21. The use of claim 19 or the process of claim 20, wherein the promotion of plant health is a prophylaxis against damage of the roots of the plants by other organisms or the improvement of the tolerance of the plant against toxic substances.

22. The use of claim 19 or the process of claim 20, wherein the promotion of plant growth results in an increase in the overall yield of the plant, an increase in quality of the plant material, an increase of the growth of the shoot, an increase in chlorophyll content of photosynthetic tissue, an increase in the growth of root and/or an increase in the overall biomass production of the plant.

23. The use of claim 19 or the process of claim 20, wherein the promotion of plant nutrition results in an increase of phosphate uptake.

24. Use of the fungus of any one of claims 1 to 6, the chlamydospore of claim 7, the mycelium of claim, 8, the culture medium of claim 11 or the filtrate of claim 12 or a combination of any of these components for promoting the regeneration of plants from plant material.

25. A process for the regeneration of plants from plant material which involves the addition of the fungus of any one of claims 1 to 6, the chlamydospore of claim 7, the mycelium of claim 8, the culture medium of claim 11 or the filtrate of claim 12 or a combination of any of these components at least at one stage of the regeneration process to the substrate on which the plant material or the developing plantlet is cultivated or to the plant material or the developing plantlet itself or to the substrate and to the plant material or plantlet.

26. The use of claim 24 or the process of claim 25, wherein the plant material comprises genetically modified cells.

27. Use of the fungus of any one of claims 1 to 6, the chlamydospore of claim 7, the mycelium of claim 8, the culture medium of claim 11 or the filtrate of claim 12 or a combination of any of these components for promoting germination of plant seeds.

28. A process for promoting germination of plant seeds comprising the step of adding the fungus of any one of claims 1 to 6, the chlamydospore of claim 7, the mycelium of claim 8, the culture medium of claim 11 or the filtrate of claim 12 or a combination of any of these components to the substrate on or in which the seed germinates or to the seed itself or to the substrate and the seed.

## Patentansprüche

1. Pilz der Gattung Piriformospora, der zumindest die folgenden Merkmale aufweist:
(a) die Hyphen sind septiert;
(b) die septalen Poren bestehen aus Doliporen mit kontinuierlichen Parenthesomen;
(c) das Myzel produziert an den Spitzen der Hyphen Chlamydosporen, die apikal entstehen;
(d) die Chlamydosporen haben eine birnenförmige Struktur; und
(e) der Pilz weist die Fähigkeit auf, mit lebenden Pflanzenwurzeln zu interagieren.

2. Pilz nach Anspruch 1, der die lebende Pflanzenwurzel sowohl intrazellulär als auch extrazellulär kolonisiert.

3. Pilz nach Anspruch 1 oder 2, **dadurch gekennzeichnet, dass** die Chlamydosporen von Myzel produziert werden, das innerhalb der lebenden Pflanzenwurzel wächst, als auch von Myzel, das außerhalb der lebenden Pflanzenwurzel wächst.

4. Pilz nach einem der Ansprüche 1 bis 3, **dadurch gekennzeichnet, dass** die Chlamydosporen etwa 14 bis 33 µm in der Länge und etwa 9 bis 20 µm in der Breite messen.

5. Pilz nach einem der Ansprüche 1 bis 4, **dadurch gekennzeichnet, dass** er in der Lage ist, auf verschiedenen natürlichen und synthetischen Medien zu wachsen.

6. Pilz nach einem der Ansprüche 1 bis 5, der DSM 11827 ist.

7. Chlamydospore des Pilzes nach einem der Ansprüche 1 bis 6.

8. Myzel des Pilzes nach einem der Ansprüche 1 bis 6.

9. Kultur des Pilzes nach einem der Ansprüche 1 bis 6.

10. Kultur nach Anspruch 9, die eine Flüssigkultur ist.

11. Kulturmedium, erhalten von der Kultur nach Anspruch 10, das frei oder im wesentlichen frei von Pilzen ist.

12. Kulturmedium nach Anspruch 11, das ein Kulturfiltrat ist.

13. Substrat, umfassend den Pilz nach einem der Ansprüche 1 bis 6, die Chlamydospore nach Anspruch 7, das Myzel nach Anspruch 8, das Kulturmedium nach Anspruch 11 oder das Filtrat nach Anspruch 12 oder eine beliebige Kombination dieser Komponenten.

14. Substrat nach Anspruch 13, das ein Flüssigsubstrat ist.

15. Substrat nach Anspruch 13, das ein Festsubstrat ist.

16. Substrat nach einem der Ansprüche 13 bis 15, das als Wachstumssubstrat für Pflanzen genutzt werden kann.

17. Zusammensetzung, umfassend den Pilz nach einem der Ansprüche 1 bis 6, die Chlamydospore nach Anspruch 7, das Myzel nach Anspruch 8, das Kulturmedium nach Anspruch 11 oder das Filtrat nach Anspruch 12 oder eine beliebige Kombination dieser Komponenten.

18. Zusammensetzung nach Anspruch 17, die ein Dünger ist.

19. Verwendung des Pilzes nach einem der Ansprüche 1 bis 6, der Chlamydospore nach Anspruch 7, des Myzels nach Anspruch 8, des Kulturmediums nach Anspruch 11 und/oder des Filtrats nach Anspruch 12 zur Förderung der Entwicklung, des Wachstums, der Gesundheit und/oder der Ernährung von Pflanzen.

20. Verfahren zur Förderung der Entwicklung, des Wachstums, der Gesundheit und/oder der Ernährung von Pflanzen, umfassend den Schritt des Zugebens des Pilzes nach einem der Ansprüche 1 bis 6, der Chlamydospore nach Anspruch 7, des Myzels nach Anspruch 8, des Kulturmediums nach Anspruch 11 oder des Filtrats nach Anspruch 12 oder einer beliebigen Kombination dieser Komponenten zu dem Substrat, auf dem die Pflanze kultiviert wird, oder zu der Pflanze selbst oder zu dem Substrat und der Pflanze.

21. Verwendung nach Anspruch 19 oder Verfahren nach Anspruch 20, wobei die Förderung der Pflanzengesundheit eine Prophylaxe gegen Beschädigung der Wurzeln der Pflanze durch andere Organismen oder die Verbesserung der Toleranz der Pflanzen gegen toxische Substanzen ist.

22. Verwendung nach Anspruch 19 oder Verfahren nach Anspruch 20, wobei die Förderung des Pflanzenwachstums zu einer Zunahme des Gesamtertrags der Pflanze, zu einer Zunahme der Qualität des Pflanzenmaterials, zu einer Zunahme des Sproßwachstums, zu einer Zunahme des Chlorophyllgehalts von photosynthetischem Gewebe, zu einer Zunahme des Wurzelwachstums und/oder zu einer Zunahme der Gesamtbiomasseproduktion der Pflanze führt.

23. Verwendung nach Anspruch 19 oder Verfahren nach Anspruch 20, wobei die Förderung der Pflanzenernährung zu einer Zunahme der Phosphataufnahme führt.

24. Verwendung des Pilzes nach einem der Ansprüche 1 bis 6, der Chlamydospore nach Anspruch 7, des Myzels nach Anspruch 8, des Kulturmediums nach Anspruch 11 oder des Filtrats nach Anspruch 12 oder einer beliebigen Kombination dieser Komponenten zur Förderung der Regeneration von Pflanzen aus Pflanzenmaterial.

25. Verfahren zur Regeneration von Pflanzen aus Pflanzenmaterial, das die Zugabe des. Pilzes nach einem der Ansprüche 1 bis 6, der Chlamydospore nach Anspruch 7, des Myzels nach Anspruch 8, des Kulturmediums nach Anspruch 11 oder des Filtrats nach Anspruch 12 oder einer beliebigen Kombination dieser Komponenten zumindest in einer Stufe des Regenerationsprozesses zu dem Substrat umfasst, auf dem das Pflanzenmaterial oder das sich entwickelnde Pflänzchen kultiviert wird, oder zu dem Pflanzenmaterial oder dem sich entwickelnden Pflänzchen selbst oder zu dem Substrat und zu dem Pflanzenmaterial oder Pflänzchen.

26. Verwendung nach Anspruch 24 oder Verfahren nach Anspruch 25, wobei das Pflanzenmaterial genetisch modifizierte Zellen umfasst.

27. Verwendung des Pilzes nach einem der Ansprüche 1 bis 6, der Chlamydospore nach Anspruch 7, des Myzels nach Anspruch 8, des Kulturmediums nach Anspruch 11 oder des Filtrats nach Anspruch 12 oder einer beliebigen Kombination dieser Komponenten zur Förderung der Keimung von Pflanzensamen.

28. Verfahren zur Förderung der Keimung von Pflanzensamen, umfassend den Schritt des Zugebens des Pilzes nach einem der Ansprüche 1 bis 6, der Chlamydospore nach Anspruch 7, des Myzels nach Anspruch 8, des Kulturmediums nach Anspruch 11 oder des Filtrats nach Anspruch 12 oder einer beliebigen Kombination dieser Komponenten zu dem Substrat, auf oder in dem der Same keimt, oder zu dem Samen selbst oder zu dem Substrat und dem Samen.

## Revendications

1. Champignon du genre Piriformospora possédant au moins les caractéristiques suivantes :
(a) les hyphes sont septés ;
(b) les pores septaux sont constitués de dolipores avec des parenthosomes continus ;
(c) le mycélium produit aux extrémités des hyphes des chlamydospores qui émergent apicalement ;
(d) les chlamydospores présentent une structure piriforme ; et
(e) le champignon a la capacité d'interagir avec les racines des plantes vivantes.

2. Champignon selon la revendication 1, qui colonise la racine d'une plante vivante à la fois intracellulairement et extracellulairement.

3. Champignon selon la revendication 1 ou 2, **caractérisé en ce que** les chlamydospores sont produites par un mycélium se développant dans la racine de la plante vivante, ainsi que par un mycélium se développant à l'extérieur de la racine de la plante vivante.

4. Champignon selon l'une quelconque des revendications 1 à 3, **caractérisé en ce que** les chlamydospores mesurent environ de 14 à 33 µm de longueur et environ de 9 à 20 µm de largeur.

5. Champignon selon l'une quelconque des revendications 1 à 4, **caractérisé en ce qu'**il est capable de se développer sur différents milieux naturels et synthétiques.

6. Champignon selon l'une quelconque des revendications 1 à 5, qui est le DSM 11827.

7. Chlamydospore du champignon selon l'une quelconque des revendications 1 à 6.

8. Mycélium du champignon selon l'une quelconque des revendications 1 à 6.

9. Culture du champignon selon l'une quelconque des revendications 1 à 6.

10. Culture selon la revendication 9, qui est une culture liquide.

11. Milieu de culture obtenu à partir de la culture selon la revendication 10, qui est exempt ou pour l'essentiel exempt de champignons.

12. Milieu de culture selon la revendication 11, qui est un filtrat de culture.

13. Substrat comprenant le champignon selon l'une quelconque des revendications 1 à 6, la chlamydospore selon la revendication 7, le mycélium selon la revendication 8, le milieu de culture selon la revendication 11 ou le filtrat selon la revendication 12 ou une combinaison quelconque de ces composants.

14. Substrat selon la revendication 13, qui est un substrat liquide.

15. Substrat selon la revendication 13, qui est un substrat solide.

16. Substrat selon l'une quelconque des revendications 13 à 15, qui peut être utilisé comme substrat de croissance pour les plantes.

17. Composition comprenant le champignon selon l'une quelconque des revendications 1 à 6, la chlamydospore selon la revendication 7, le mycélium selon la revendication 8, le milieu de culture selon la revendication 11 ou le filtrat selon la revendication 12 ou une combinaison quelconque de ces composants.

18. Composition selon la revendication 17, qui est un fertilisant.

19. Utilisation du champignon selon l'une quelconque des revendications 1 à 6, de la chlamydospore selon la revendication 7, du mycélium selon la revendication 8, du milieu de culture selon la revendication 11 et/ou du filtrat selon la revendication 12 pour favoriser le développement, la croissance, la santé et/ou la nutrition des plantes.

20. Procédé pour favoriser le développement, la croissance, la santé et/ou la nutrition des plantes comprenant l'étape consistant à ajouter le champignon selon l'une quelconque des revendications 1 à 6, la chlamydospore selon la revendication 7, le mycélium selon la revendication 8, le milieu de culture selon la revendication 11 ou le filtrat selon la revendication 12
ou une combinaison quelconque de ces composants au substrat sur lequel la plante est cultivée ou à la plante elle-même ou au substrat et à la plante.

21. Utilisation selon la revendication 19 ou procédé selon la revendication 20, dans lequel l'effet favorisant la santé de la plante est une prophylaxie contre les lésions des racines de la plante par d'autres organismes ou l'amélioration de la tolérance de la plante aux substances toxiques.

22. Utilisation selon la revendication 19 ou procédé selon la revendication 20, dans lequel l'effet favorisant la croissance de la plante entraîne une augmentation du rendement global de la plante, une augmentation de la qualité du matériel de la plante, une augmentation de la croissance de la pousse, une augmentation de la teneur en chlorophylle des tissus photosynthétiques, une augmentation de la croissance de la racine et/ou une augmentation de la production de biomasse globale de la plante.

23. Utilisation selon la revendication 19 ou procédé selon la revendication 20, dans lequel l'effet favorisant la nutrition de la plante entraîne une augmentation de l'assimilation des phosphates.

24. Utilisation du champignon selon l'une quelconque des revendications 1 à 6, de la chlamydospore selon la revendication 7, du mycélium selon la revendication 8, du milieu de culture selon la revendication 11 ou du filtrat selon la revendication 12 ou d'une combinaison quelconque de ces composants pour favoriser la régénération des plantes à partir d'un matériel de plante.

25. Procédé pour la régénération des plantes à partir d'un matériel de plante qui comprend l'addition du champignon selon l'une quelconque des revendications 1 à 6, de la chlamydospore selon la revendication 7, du mycélium selon la revendication 8, du milieu de culture selon la revendication 11 ou du filtrat selon la revendication 12 ou d'une combinaison quelconque de ces composants au moins à une étape du procédé de régénération, au substrat sur lequel le matériel de plante ou la plantule en développement est cultivé, ou au matériel de plante lui-même ou à la plantule en développement elle-même, ou au substrat et au matériel de plante ou à la plantule.

26. Utilisation selon la revendication 24 ou procédé selon la revendication 25, dans lequel le matériel de plante comprend des cellules génétiquement modifiées.

27. Utilisation du champignon selon l'une quelconque des revendications 1 à 6, de la chlamydospore selon la revendication 7, du mycélium selon la revendication 8, du milieu de culture selon la revendication 11 ou du filtrat selon la revendication 12 ou d'une combinaison quelconque de ces composants pour favoriser la germination des graines de plantes.

28. Procédé pour favoriser la germination des graines de plantes comprenant l'étape consistant à ajouter le champignon selon l'une quelconque des revendications 1 à 6, la chlamydospore selon la revendication 7, le mycélium selon la revendication 8, le milieu de culture selon la revendication 11 ou le filtrat selon la revendication 12 ou une combinaison quelconque de ces composants, au substrat sur ou dans lequel la graine germe, ou à la graine elle-même, ou au substrat et à la graine.
